# EUROPEAN PATENT APPLICATION

(11) **EP 4 129 331 A1**
(43) Date of publication of application: **08.02.2023**
(21) Application number: 21780982.1
(22) Date of filing: 31.03.2021
(51) Int. Cl.: A61K 39/155, A61K 39/12, C12N 1/21, A61P 37/04, A61P 31/12, A61P 31/14, A61P 11/00

(54) **NOVEL USE OF BCG IMMUNOGENIC FORMULATION EXPRESSING A HUMAN RESPIRATORY SYNCYTIAL VIRUS PROTEIN AGAINST HMPV IN CATTLE**

(30) Priority: 31.03.2020 CL 20200857
(71) Applicant: Pontificia Universidad Católica De Chile, 8331150 Santiago (CL)
(72) Inventor: KALERGIS, Alexis, Santiago, 8331150 (CL); BUENO, Susan, Santiago, 8331150 (CL); GONZÁLEZ, Pablo, Santiago, 8331150 (CL)
(74) Representative: Elzaburu S.L.P.
(86) International application number: PCT/CL2021/050022
(87) International publication number: WO 2021/195797

(57) **Abstract**

The invention relates to the novel use of an immunogenic formulation containing Bacillus Calmette-Guerin (BCG) in a concentration between 10⁴-10⁹ bacteria, which recombinantly expresses at least one protein or immunogenic fragment of the human Respiratory Syncytial Virus (hRSV, *human orthopneumovirus*)*,* stabilized in a pharmaceutically acceptable saline buffer solution to prevent, treat, or attenuate bRSV infections in cattle.

## Description

### FIELD OF INVENTION

An immunogenic formulation useful for preparing a vaccine against bovine respiratory syncytial virus (bRSV, *bovine orthopneumovirus*) is presented, where this formulation comprises at least one attenuated strain of *Mycobacterium,* preferably a Bacillus Calmette-Guérin (BCG) strain, which recombinantly expresses one or more proteins or immunogenic fragments of hRSV.

### BACKGROUND OF THE INVENTION

Human Respiratory Syncytial Virus (hRSV) infection is one of the leading causes of acute infection in the lower respiratory tract in infants and children under 5 years of age worldwide. It is not yet clear which type of immune response is most effective for the elimination and prevention of the hRSV infection. Natural hRSV infection induces a mixed response between Th 1 and Th2, resulting in harmful lung inflammation and does not induce a long-lasting memory response. It is postulated that inducing a more balanced Th1-type immune response against hRSV will result in a better disease outcome and long-lasting immune memory.

*Mycobacterium bovis* Bacillus Calmette-Guerin (BCG) is the *M. bovis* vaccine strain administered worldwide for the prevention of tuberculosis. *M. bovis* BCG induces a potent and sustained Th1-type immune response. It has an excellent safety profile and is routinely administered to infants and children under 5 years of age. The inventors developed a recombinant strain of BCG expressing the nucleoprotein (N) of the hRSV nucleocapside (rBCG-N-hRSV), which is protected in the WO2009/039178 patent family.

Vaccination with rBCG-N-hRSV induces specific cellular and humoral immunity against hRSV, protects mice against lung damage associated with hRSV, and reduces viral load in lung tissues after challenge. This is the first vaccine of its kind in development for use in neonates, and has the potential to protect infants from both hRSV infection and tuberculosis. This vaccine is in its final stages for approval for use in humans, especially neonates.

On the other hand, bovine RSV (bRSV) is a virus distinct from hRSV, although genetically related to hRSV and represents an important cause of morbidity in young cattle (bovines). bRSV infection in calves shows many similarities to hRSV infection in newborn humans, including similar age-related susceptibility, seasonal periodicity, macroscopic and microscopic pathology, and innate and adaptive immune responses. Neonatal calves have a particularly high risk of severe bRSV infection due to their immature immune system, similar to that which occurs in infant humans.

Surprisingly, the inventors have found that the vaccine developed for hRSV (rBCG-N-hRSV) gives neonatal calves protection against bRSV infection.

Due to the differences between the two viruses, it is not obvious or predictable that the vaccine developed based on the virus that infects humans could provide protection for cattle against bRSV. As we will explain in detail later, the vaccine of the invention is a formulation containing the Bacillus Calmette-Guérin (BCG) strain, in a concentration between 1×10⁴ to 1×10⁹ colony-forming units per dose, expressing at least one protein or immunogenic fragment of hRSV.

In the state of the art some vaccines against bRSV have been developed that differ from the vaccine used in the present invention, since it was originally developed for humans and against hRSV.

For example, Zhang, Baoshan, et al. (Protection of calves by a prefusion-stabilized bovine RSV F vaccine. npj Vaccines, 2017, vol. 2, no 7, p. 1-11), disclose a vaccine against bRSV that uses bRSV F protein as an anti-viral component. According to the inventors, they relied on a vaccine formulated for humans with the hRSV F protein, and employed a combination of structure-based design, antigenic a characterization and X-ray crystallography to translate hRSV F stabilization in the bovine context. This publication discloses a DS2-stabilized version of the Fprotein of bovine respiratory syncytial virus with covalently fused subunits, elimination of the fusion peptide and emulating the pre-fusion conformation, achieved by mutations. This protein was recognized in the laboratory by specific antibodies obtained for bRSV F protein prior to fusion. The results show thatbRSV F immunogens stabilized with DS2 may induce highly protective immunity against RSV in a native host with implications for the prevention of bRSV in calves.

As can be seen, for the authors of this work, the vaccine developed for humans must be adapted to be used in cattle, and it was the strategy they followed, contrary to what was done in the present invention.

### DESCRIPTION OF THE FIGURES

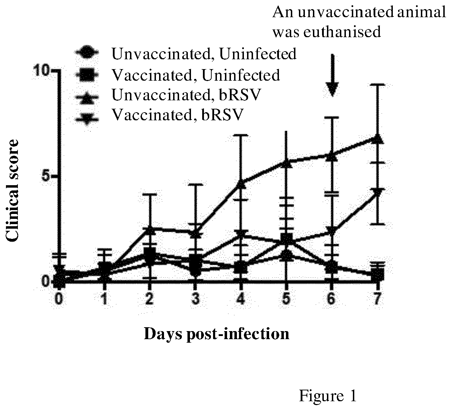

***Figure 1******. Reduction of symptoms of bRSV disease in calves vaccinated with rBCG-N-*hRSV.** Results are shown from the following groups: Control groups unvaccinated, uninfected; vaccinated with rBCG-N-hRSV, not infected; unvaccinated, infected with bRSV; vaccinated with rBCG-N-hRSV and infected with bRSV. The calves were infected with the bRSV 375 strain by aerosol inoculation. Calves in all four groups were monitored daily by a blind observer and assigned a clinical score using the criteria described in the examples. A calf in the control group infected with bRSV, unvaccinated; was euthanized on day 6 due to signs of severe illness. The data represents ± SEM. * p <0.05 as determined by 2-way ANOVA with repeated measures, followed by the Sidak multiple comparison test.

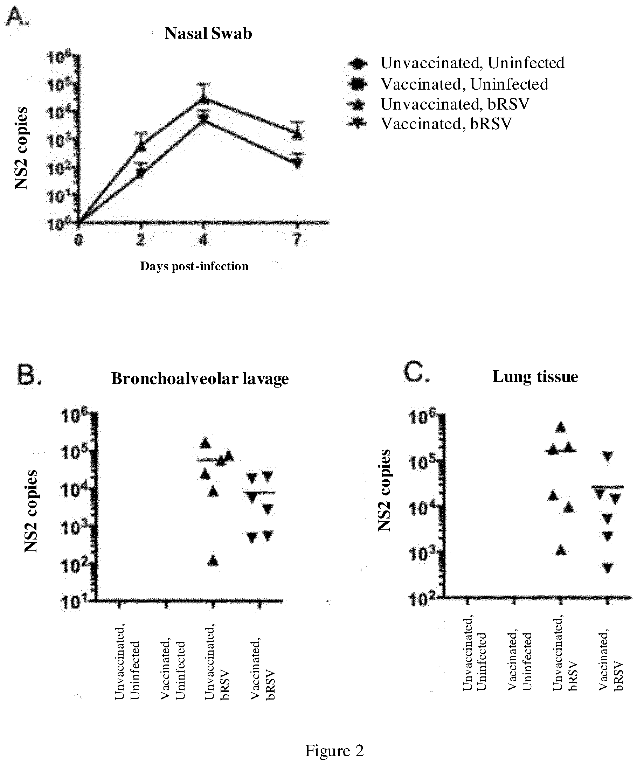

***Fig 2******. Elimination of virus and pulmonary viral load in the nasal swabs, BAL and lung.*** (A)Nasal swabs were collected on days 0, 2, 4 and 7 after bRSV infection. The samples were divided and analyzed for virus isolation. (Table 1) and quantification of viruses by qPCR for the bRSV Gene NS2. Broncoralveolar lavage fluid (BAL) (B) and lung tissue samples (C) were also analyzed by qPCR for the NS2 gene. Viral NS2 copy numbers were calculated using standard curves. Lung tissue and BAL samples were normalized by the S9 gene, to correct differences in the input material. Data points represent ± SEM for each group. No viral NS2 was detected in a sample of uninfected control calves. No significant differences were observed between unvaccinated and rBCG-N-hVRS vaccinated animals.

### DETAILED DESCRIPTION OF THE INVENTION

The present invention consists of an immunogenic formulation that can be used for the preparation of vaccines that induce protection against infections caused by bovine Respiratory Syncytial Virus (bRSV) or that attenuate the pathologies caused by this virus in cattle, specifically in calves. The vaccine of the invention contains live recombinant attenuated strains of *Mycobacterium,* preferably Bacillus Calmette-Guérin (BCG), for example the Danish or Pasteur BCG strains that express in a recombinant or heterologous way one or more proteins or immunogenic fragments of hRSV. The vaccine of the invention comprises between 1×10⁴ - 1×10⁹ colony-forming units (CFU) of the strains described by dose, and can be kept prior to administration, in lyophilized form or in a cold stabilizing saline solution.

Examples of stabilized vaccines in appropriate solutions for the immunological formulations of the invention are:
- Sauton Dilute Solution SSI (125 µg MgSO₄, 125 µg K₂HPO₄, 1 mg L-asparragine, 12.5 µg ferric ammonium citrate, 18.4 mg 85% glycerol, 0.5 mg citric acid, in 1 ml of H₂O) at 4°C,
- PBS (137 mM NaCl; 2.7 mM KCl; 4.3 mM Na₂HPO₄; 1.47 mM KH₂PO₄, pH 7.4) supplemented with Tween 80 0.02% and Glycerol 20% at -80°C or
- Solution volume: volume of lactose 25% and Proskauer and Beck Medium supplemented with glucose and Tween 80 (PBGT: 0.5 g asparragine; 5.0 g phosphate monopotassium; 1.5 g magnesium citrate; 0.5 g potassium sulfate; 0.5 ml Tween 80 and 10.0 g glucose per liter of distilled water) lyophilized and preserved in the temperature range between 4°C and 25°C.

The attenuated recombinant *Mycobacterium* bacteria of the immunogenic formulation of the present invention contain genes encoding for at least one protein, or immunogenic fragment of hRSV of the hRSV A or hRSV B subtypes, or both. The genome of the Respiratory Syncytial Virus has been previously described in the GeneBank database, access numbers NC_001803 and NC_001781.

The genes that code for at least one protein, or immunogenic fragment of hRSV of the hRSV A or hRSV B subtypes, or both, of the present invention, have at least 80% identity with the genes described in said genebank disclosed sequences, access numbers NC_001803 and NC_001781.

The proteins, or immunogenic fragments of hRSV correspond to the proteins NS1, NS2, N, P, M, SH, M2 (ORF1), M2 (ORF2), L, For G of RSV. In a preferred formulation the immunogenic proteins or fragments correspond to N, P, M, SH, M2 (ORF1), M2 (ORF2), L, For G of hRSV.

To obtain the recombinant strains of the invention, the genes that code for these proteins or their immunogenic fragments, are inserted into a plasmid, which is incorporated into the bacterium by any available technique. In one formulation, a plasmid is used that is incorporated into the bacterium by electrotransformation, and is integrated into the bacterial genome by the action of mycobacteriophage integrases. These genes can also be inserted into extracchromosomal plasmids, which are incorporated into *Mycobacterium* by electrotransformation, and maintained extrachromosomally in the bacterium. These genes can be in one or more copies, and their expression is commanded by endogenous BCG promoters, constitutive or inducible, for example the promoter of the *hsp60* gene and the promoter of the *acr* gene respectively. These proteins, or immunogenic fragments of hRSV, can be expressed by BCG or other attenuated strains of *Mycobacterium,* in a soluble-cytoplasmic form, secreted extracellularly or as proteins bound to the cell membrane thanks to the use of respiratory syncytial virus genes, or immunogenic fragments, with DNA sequences that code for peptides that function as destination signals of proteins to the different bacterial compartments, for example the N-terminal sequence of the gene for the alpha-antigen for extracellular secretion and the N-terminal sequence of the gene for the 19 kD protein for membrane-bound proteins.

The immunogenic formulation that is disclosed in the present invention can be used in conjunction with immunogenic formulations containing one or more attenuated strains of *Mycobacterium* or BCG and differing in the immunogenic proteins of hRSV that they express, as well as in the location of the genes (inserted into the genome or extrachromosomal), the number of copies of the gene of the protein, the promoter that induces the expression of the protein, or the destination of the protein or immunogenic fragments of hRSV (soluble-cytoplasmic, extracellularly secreted or proteins attached to cell membrane).

The inventors have shown that the vaccines of the invention induce a Th1-type immune response, which includes both antibody-producing B lymphocytes of IgG2a isotype, as an effective response of IFN-γ-producing T lymphocytes. This guarantees a humoral protection against these viruses and an efficient cellular response that enhances both the effectiveness and applicability of the immunogenic formulation of the invention.

The vaccine of the invention can be applied to the individual subdermally, in conjunction with a buffer or physiological saline solution.

The immunogenic formulation of the invention can be used to vaccinate cattle, especially those thathave not had previous contact with bRSV, in order to confer protection against this virus or attenuate the pathology caused by said virus in the future.

The following examples extend to immunological formulations containing a recombinant strain of attenuated *Mycobacterium* expressing the proteins NS2, N, P, M, SH, M2 (ORF1), M2 (ORF2), L, F or G of hRSV, as well as all combinations of these formulations. Likewise, the examples are extended to immunological formulations containing one or more recombinant strains of attenuated BCG; where these recombinant bacteria contain genes that encode proteins, or immunogenic fragments of hRSV that are inserted in the bacterial genome or in extrachromosomal plasmids, in one or more copies, and their expression is commanded by endogenous or exogenous promoters, constitutive or inducible, expressed, soluble-cytoplasmic, secreted extracellularly or as proteins bound to the cell membrane.

As will be seen in the examples, the inventors tested the vaccine on calves which were subsequently challenged with bRSV. Taken together, the results of clinical disease, pulmonary pathology, and BAL cytology (bronchial lavages) demonstrate that calves vaccinated with rBCG-N-hRSV do not develop any symptoms of vaccine-potentiated disease, suggesting that rBCG-N-hRSV is safe for use in neonatal cattle. In addition, we observed significant reductions in clinical disease between vaccinated and unvaccinated animals and reduced neutrophil infiltration into the lungs, demonstrating that rBCG-N-hRSV may offer protection against bRSV infection in neonatal calves.

The following examples are illustrative, and not limiting, of an application of the invention.

### EXAMPLES

### Example I: Immunogenic formulation consisting of 10⁸ bacteria of the recombinant BCG Danish strain for the N gene of hRSV subtype A.

The gene is inserted in a copy in the genome of the bacterium under the regulation of the constitutive endogenous promoter *hsp60* of BCG and the expression of the protein is cytoplasmic. The immunogenic formulation can be found in a Sauton Dilute Solution SSI (125 µg MgSO₄, 125 µg K₂HPO₄, 1 mg L-asparragine, 12.5 µg ferric ammonium citrate, 18.4 mg 85% glycerol, 0.5 mg citric acid in 1 ml of H2O) at -80°C. Also the formulation can be found in a solution of PBS (137 mM NaCl; 2.7 mM KCl; 4.3 mM Na₂HPO₄; 1.47 mM KH₂PO₄, pH 7.4), supplemented with Glycerol 20% and Tween 80 0.02% at a final concentration of 10⁸ bacteria per 100 µl and preserved at -80°C. Similarly, the strains can be resuspended in a solution volume: lactose volume 25% and Proskauer and Beck medium supplemented with glucose and Tween 80 (PBGT: 0.5 g asparragine; 5.0 g monopotasium phosphate; 1.5 g magnesium citrate; 0.5 g potassium sulfate; 0.5 ml Tween 80 and 10.0 g glucose per liter of distilled water) to then be lyophilized and preserved at 25°C.

The BCG Danish strain was transformed by electrotransformation with the pMV361/N plasmid, derived from the pMV361 plasmid, which is inserted only once into the bacterium's genome. This plasmid contains the gene encoding the N protein of hRSV subtype A, which is expressed under the endogenous promoter and constitutive of the BCG *hsp60* gene. The resulting recombinant colonies were grown at 37°C in Middlebrock 7H9 culture medium supplemented to OD₆₀₀ₙₘ=1, centrifuged at 4,000 rpm per 20 min (eppendorf rotor model 5702/R A-4-38) and resuspended in PBS solution (137 mM NaCl; 2.7 mM KCl; 4.3 mM Na₂HPO₄; 1.47 mM KH₂PO₄, pH 7.4), supplemented with Glycerol 20% and Tween 80 0.02% at a final concentration of 10⁸ bacteria per 100 µl and preserved at -80°C. Similarly, the strains can be resuspended in a solution volume: lactose volume 25% and Proskauer and Beck medium supplemented with glucose and Tween 80 (PBGT: 0.5 g asparragine; 5.0 g monopotasiumphosphate; 1.5 g magnesium citrate; 0.5 g potassium sulfate; 0.5 ml Tween 80 and 10.0g glucose per liter of distilled water) to then be lyophilized and preserved at 25°C. By Western blot, with the use of antibodies to RSV's N protein, the inventors observed that this strain of BCG recombinantly expresses the N protein of RSV subtype A in the cytoplasm.

### ExampleII: Protection conferred by BCG-N ofhRSV against bRSV infections in cattle.

To determine the protection afforded by the rBCG-N-hVRS vaccine against bRSV in cattle, a total of 19 newborn calves, colostrum fed, Holstein cattle were evaluated. The calves were divided into 4 groups:
1) Unvaccinated, uninfected controls (2 animals)
2) rBCG-N-hRSV vaccinated, uninfected (5 animals)
3) unvaccinated, infected with bRSV (6 animals)
4) rBCG-N-hRSV vaccinated, infected with bRSV (6 animals)

At 5 days of age, calves in groups 2 and 4 were vaccinated subcutaneously on the right side of the neck with 10⁶ CFU of rBCG-N-hRSV suspended in 500 µl of sterile saline. The control group calves remained unvaccinated. Two weeks after primary vaccination, calves in groups 2 and 4 received a vaccine booster on the right side of the neck with 10⁶ CFUs of rBCG-N-hRSV suspended in 500 µl of sterile saline.

For the challenge, the animals were inoculated 5 ml per intranasal aerosol with the bRSV 375 strain, at a concentration of 10⁴ TCID₅₀/ mL of bRSV 375 (TCID: *Tissue culture infectious doses*)*.* Dilution of the virus to which it infects 50% of the cells in culture.

After infection, all animals were monitored daily for body temperature and clinical signs including appetite, respiratory rate, cough, runny nose and eye discharge, and lung auscultation.

A trained observer who acted as a blind rated the calves once a day for clinical illness. The scorecard assigns numbers (0-3) based on fever and severity of clinical signs. For our scorecard, we included two additional categories for expiratory exertion (0 = effortless to 3 = significant exertion) and lung sounds by auscultation (0 = clear, 1 = wheezing and crackling). The scores for each category are added together to determine the overall clinical score, with a maximum possible score of 18. In particular, animals that receive a score of 3 in more than 3 categories for> 72 hours are euthanized to minimize unnecessary pain and distress.

Nasal fluid samples were collected at weekly intervals after vaccination, and on days 0, 2, 4 and 6 after the challenge with bRSV, using sterile polyurethane sponge pieces moistened with 1 ml of sterile saline. These were inserted into the calf's nostril for 5-10 minutes. The sponges were then removed, placed in a tube, and an additional 1 ml of sterile saline was added. The fluid was recovered from each sponge by squeezing into the lumen of a 5 ml syringe. The resulting nasal fluid was divided into aliquot parts and frozen at -80 ° C for later use.

Peripheral blood mononuclear cells (PBMCs) and serum were collected at regular intervals after vaccination and on days 3 and 7 after exposure to bRSV.

Calves in group 3 (unvaccinated, infected with bRSV) presented significant clinical signs that began on days 4-5 after infection (Fig. 1), including fever, lethargy, runny nose, and dyspnea. One animal was slaughtered on day 6 after infection due to the severity of the disease developed. Calves receiving the rBCG-N-hRSV vaccine (group 4) also developed signs of bRSV infection; however, the bRSV-associated disease had significantly lower symptoms compared to group 3 calves (Fig. 1). Importantly, we observed no signs of vaccine-boosted disease in any of the vaccinated calves as a control, nor in the vaccines and those challenged with bRSV (Fig. 1).

As indicated, nasal swabs were collected from each animal on days 0, 2, 4 and 7 after infection. On day 7 after the challenge, the animals were euthanized and samples of lung tissue and bronchoalveolar lavage fluid were obtained. The samples were processed for virus isolation and analyzed using qPCR for the NS2 bRSV gene. As shown in Table 1, no virus was detected from the nasal swabs of any calf before the challenge against bRSV. After bRSV infection, the virus was isolated from the nasal swabs of most animals in groups 3 and 4 throughout infection, regardless of vaccination status. bRSV was isolated from lung tissue samples and BALs from calves 5/6 and 6/6 in group 3 (unvaccinated), respectively; and from 4/6 calves in group 4 (vaccinated with rBCG-N-hRSV) on day 7 after infection. bRSV was not detected in calves in groups 1 or 2 at any time.

**Table 1. Virus isolation results from nasal, BAL fluid, and lung tissue samples.**

| Group | Nasal swabs | | | | BAL | Lung |
|---|---|---|---|---|---|---|
| | Day0 (Challenge) | Day2 p.i. | Day4 p.i. | Day7 p.i. (necropsy) | Day7 p.i. | Day 7 p.i. |
| Unvaccinated, uninfected | 0/2 | 0/2 | 0/2 | 0/2 | 0/2 | 0/2 |
| Vaccinated, uninfected | 0/5 | 0/5 | 0/5 | 0/5 | 0/5 | 0/5 |
| Unvaccinated, bRSV infected | 0/6 | 5/6 | 5/6 | 4/6 | 5/6 | 6/6 |
| Vaccinated, bRSV infected | 0/6 | 4/6 | 4/6 | 3/6 | 4/6 | 4/6 |

Additionally, a quantitative analysis by qPCR for the NS2 gene of bRSV was performed, which did not reveal significant differences in the amount of virus present in the nasal swabs at any time after infection between groups 3 and 4 (Fig. 2A). Similarly, we observed no significant differences in viral load in BALs or lung tissue between groups 3 and 4 (Fig. 2B and 2C). This is indicative that treatment with the vaccine does not eliminate the infection, but it does help the immune system control the development and severity of the disease.

## Claims

1. Use of an immunogenic formulation containing the Bacillus Calmette-Guerin (BCG) strain, in a concentration between 10⁴-10⁹ bacteria, expressing at least one protein or immunogenic fragment of the human Respiratory Syncytial Virus (hRSV), in a pharmaceutically acceptable buffered saline solution **CHARACTERIZED in that** it serves to prepare a vaccine useful for preventing, treating, or attenuating respiratory syncytial virus (bRSV) infections in cattle.

2. Use of claim 1 where the protein or immunogenic fragment of hRSV corresponds to hRSV subtypes A or hRSV subtypes B or to both subtypes **CHARACTERIZED in that** it serves to prepare a vaccine useful for preventing, treating, or attenuating bovine respiratory syncytial virus (bRSV) infections in cattle.

3. Use of claim 2 where the hRSV immunogenic protein or fragment belongs to the group consisting of the proteins NS2, N, P, M, SH, M2 (ORF1), M2 (ORF2), L, F or G **CHARACTERIZED in that** it serves to prepare a vaccine useful for preventing, treating, or attenuating bovine respiratory syncytial virus (bRSV) infections in cattle

4. Use of claim 3 where the genes encoding the proteins or immunogenic fragments of hRSV are inserted into the genome of *Mycobacterium,* preferably BCG or in extracromosomal plasmids, in one or more copies **CHARACTERIZED in that** it serves to prepare a vaccine useful for preventing, treating, or attenuating bovine respiratory syncytial virus (bRSV) infections in cattle.

5. Use of claim 4 where the expression of protein genes are commanded by endogenous or exogenous promoters of *Mycobacterium* BCG, constitutive or inducible **CHARACTERIZED in that** it serves to prepare a vaccine useful for preventing, treating, or attenuating bovine respiratory syncytial virus (bRSV) infections in cattle.

6. Use of claim 1 where immunogenic proteins or fragments of hRSV can be expressed by BCG in a soluble-cytoplasmic form, secreted extracellularly or as proteins bound to cell membrane **CHARACTERIZED in that** it serves to prepare a vaccine useful for preventing, treating, or attenuating bovine respiratory syncytial virus (bRSV) infections in cattle.

7. Use of claim 3 where the protein or immunogenic fragment of hRSV corresponds to the protein N of hRSV subtypes A **CHARACTERIZED in that** it serves to prepare a vaccine useful for preventing, treating, or attenuating bovine respiratory syncytial virus (bRSV) infections in cattle.

8. Use of claim 6 where the formulation can be used in conjunction with other immunogenic formulations, expressing proteins or immunogenic fragments other than NS2, N, P, M, SH, M2 (ORF1), M2 (ORF2), L, F or G of VRSh **CHARACTERIZED in that** it serves to prepare a vaccine useful for preventing, treating, or attenuating infections of bovine respiratory syncytial virus (bRSV) in cattle.

9. Use of claim 6 where the formulation can be used in conjunction with other immunogenic formulations, which differ in the form expressed by immunogenic proteins or fragments, soluble, soluble-cytoplasmic, extracellularly secreted or proteins bound to cell membrane **CHARACTERIZED in that** it serves to prepare a vaccine useful for preventing, treating, or attenuating infections of bovine respiratory syncytial virus (bRSV) in cattle.

10. Use of claim 6 where the formulation can be used in conjunction with other immunogenic formulations or differing in the number of copies of the genes of hRSV proteins or immunogenic fragments, as well as in the way in which the expression of hRSV proteins or immunogenic fragments are controlled, constitutive or inducible, and further differ in the destination of hRSV protein or immunogenic fragment **CHARACTERIZED in that** it serves to prepare a vaccine useful for preventing, treating, or attenuating bovine respiratory syncytial virus (bRSV) infections in cattle.

11. Use of claim 6 where the formulation can be stabilized by freezing, freeze-drying or saline buffer for preservation prior to use **CHARACTERIZED in that** it serves to prepare a vaccine useful for preventing, treating, or attenuating bovine respiratory syncytial virus (bRSV) infections in cattle.

12. Use of claim 11 where the formulation can be administered subdermally in acceptable physiological saline **CHARACTERIZED in that** it serves to prepare a vaccine useful for preventing, treating, or attenuating bovine respiratory syncytial virus (bRSV) infections in cattle.
